# EUROPÄISCHE PATENTANMELDUNG

(11) **EP 2 243 774 A1**
(43) Veröffentlichungstag der Anmeldung: **27.10.2010**
(21) Anmeldenummer: 10163579.5
(22) Anmeldetag: 26.05.2004
(51) Int. Cl.: C07D 295/205, C07C 311/10, C07D 295/215, A61K 31/495, A61K 31/18, A61P 35/00

(54) **Hydroxyamidin- und Hydroxyguanidin-Verbindungen als Urokinase-Hemmstoffe**

(30) Priorität: 26.05.2003 DE 10323898
(62) Teilanmeldung aus: 04734821.4
(71) Anmelder: Wilex AG, 81675 München (DE)
(72) Erfinder: Sperl, Stefan, 81549, München (DE); Bürgle, Markus, 81369, München (DE); Schmalix, Wolfgang, 82194, Gröbenzell (DE); Wosikowski, Katja, 85586, Poing (DE); Clement, Bernd, 24106, Kiel (DE)
(74) Vertreter: Weiss, Wolfgang

(57) **Zusammenfassung**

Die vorliegende Erfindung betrifft neue Verbindungen zur Hemmung des Urokinase-Plasminogen-Aktivators (uPA) mit hoher Bioverfügbarkeit und oraler Verabreichbarkeit sowie deren Verwendung als therapeutische Wirkstoffe zur Behandlung von Urokinase oder/und Urokinase-Rezeptor assoziierten Erkrankungen, wie z.B. Tumore und Metastasierung. Die Erfindung betrifft insbesondere Hydroxyamidin- oder Hydroxyguanidingruppen enthaltende Verbindungen. 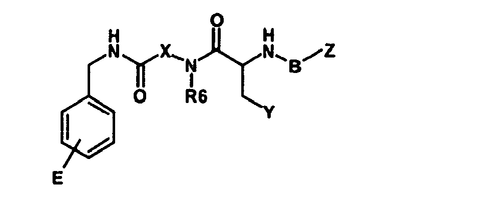 worin
E eine Gruppe aus

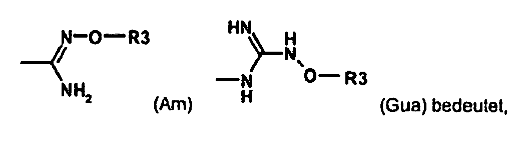

## Beschreibung

Die vorliegende Erfindung betrifft neue Verbindungen zur Hemmung des Urokinase-Plasminogen-Aktivators (uPA) mit hoher Bioverfügbarkeit und oraler Verabreichbarkeit sowie deren Verwendung als therapeutische Wirkstoffe zur Behandlung von Urokinase oder/und Urokinase-Rezeptor assoziierten Erkrankungen, wie z.B. Tumore und Metastasierung. Die Erfindung betrifft insbesondere Hydroxyamidin- oder Hydroxyguanidingruppen enthaltende Verbindungen.

Der Plasminogenaktivator vom Urokinase-Typ (uPA) spielt eine Schlüsselrolle bei der Tumorinvasion und Metastasenbildung (Schmitt et al., J. Obst. Gyn. 21 (1995), 151-165). uPA wird in den verschiedensten Arten von Tumorzellen exprimiert (Kwaan, Cancer Metastasis Rev. 11 (1992), 291-311) und bindet an den Tumor-assoziierten uPA-Rezeptor (uPAR), wo die Aktivierung von Plasminogen zu Plasmin stattfindet. Plasmin ist in der Lage, verschiedene Komponenten der extrazellulären Matrix (ECM), wie Fibronectin, Laminin und Kollagen Typ IV, abzubauen. Es aktiviert auch einige andere ECM-abbauende Enzyme, insbesondere Matrix-Metalloproteinasen. Hohe Mengen an Tumor assoziierten uPA korrelieren mit einem höheren Metastasierungsrisiko für Krebspatienten (Harbeck et al., Cancer Research 62 (2002), 4617-4622). Eine Hemmung der proteolytischen Aktivität von uPA ist daher ein guter Ansatzpunkt für eine anti-metastatische Therapie.

Einige aktive und selektive Urokinase-Inhibitoren sind schon beschrieben. So werden uPA-Inhibitoren des Benzamidin-Typs in EP 1 098 651, uPA-Inhibitoren des Arylguanidin-Typs in WO 01/96286 und WO 02/14349 offenbart. Ein gemeinsames Merkmal dieser synthetischen Inhibitoren ist ein basischer Rest bestehend aus einer Amidino- oder/und Guanidino-Gruppe. Die bekannten Urokinase-Inhibitoren weisen jedoch den Nachteil auf, dass sie bei oraler Anwendung schlecht resorbiert werden und somit bei dieser Applikationsart nur geringe pharmakologische Wirkung im Körper ausüben können. Pharmazeutische Zubereitungen werden daher zumeist einmal, jedoch bis zu zweimal wöchentlich intravenös über einen Zeitraum von mehreren Stunden dem Patienten verabreicht. Dies ist mit einer hohen Belastung des Patienten verbunden, da dies mit erheblichem Zeitaufwand und häufigen Klinikbesuchen verbunden ist und setzt ein hohes Maß an Kooperation des Patienten voraus.

Bei intravenöser Applikation besteht zudem das Risiko von Infektionen und vor allem bei paravasal austretendem Infusat kann es zu starken lokalen Reizungen bis hin zu Gewebsnekrosen kommen, welche langwierige Nachfolgebehandlungen und -beobachtung erforderlich machen.

Intramuskuläre und subkutane Applikationswege bieten auch keine Vorteile, da hier häufig starke Schmerzen an den Einstichstellen sowie Reizungen bis hin zu Gewebsnekrosen auftreten können, die ebenfalls einer langwierigen Folgebehandlung bedürfen.

Wie schon dargelegt, zeigen die Amidin- und Guanidin-enthaltenden Urokinase-Inhibitoren bei oraler Anwendung eine nur geringe pharmakologische Wirkung. Eine Voraussetzung für den therapeutischen Effekt eines Wirkstoffs stellt dessen Bioverfügbarkeit dar. So muss nach oraler Gabe eine Aufnahme aus dem Magen-Darm-Trakt erfolgen. Ein wichtiger Mechanismus für einen solchen Membrandurchtritt ist dabei die passive Diffusion (Gangvar S. et al., DDT (1997) 148-155). In der Literatur wurde teilweise angenommen, dass die Lipophilie eines Wirkstoffs eine wichtige Rolle für die passive Diffusion über die Membranbarrieren des Magen-Darm-Trakts spielt. So wird in EP 0 708 640 eine Modifizierung von Amidinfunktionen zu Amidoxim, Amidoximester und Oxadiazol für antihelmintisch wirkenden Pentamidine beschrieben, wobei bevorzugt die Amidoximester und Oxadiazol als geeignete Modifikationen eingesetzt werden.

Andererseits wurde jedoch gezeigt, dass der Grad der Lipophilie alleine nicht ausreichend ist (Hansch et al., J. Am. Chem. Soc. 86 (1964) 1616-1626) und eine Erhöhung der Lipophilie der Verbindungen keinen geeigneten Parameter zur Vorhersage für den Membrandurchtritt darstellt. So konnte eine direkte Relation zwischen Lipophilie und Membranpermeation nicht festgestellt werden (Conradi et al., Pharm. Res. 9 (1992) 435-439).

Die Erhöhung der Lipophilie kann daher in Einzelfällen die Membranpermeation erhöhen was jedoch nicht notwendigerweise eine erhöhte orale Bioverfügbarkeit zur Folge hat. So führt für Argatroban die Umwandlung des basischen Rests in das Amidoxim als Prodrug zu verbesserter Permeabilität, aber zusätzlich zum Verlust der Aktivität (Rewinkel, Adang Cur. Pharm. Design 5 (1999) 1043-1075). Es ist somit nicht ohne Weiteres vorhersehbar, ob und welche Modifikationen den Membrandurchtritt im Magen-Darm-Trakt bei einem Wirkstoff verbessern können. Noch weniger ist vorhersehbar, welchen Einfluss diese Modifikationen auf die pharmazeutischen Eigenschaften des Wirkstoffs haben können.

Eine Aufgabe der vorliegenden Erfindung bestand in der Bereitstellung von neuen Arzneimitteln zur Hemmung von Urokinase, die bei oraler Verabreichung eine im Organismus deutlich erhöhten Bioverfügbarkeit und Aktivität besitzen.

Diese Aufgabe wird erfindungsgemäß gelöst durch ein Arzneimittel, das als Wirkstoff eine oder mehrere Verbindungen der allgemeinen Formel I oder/und II 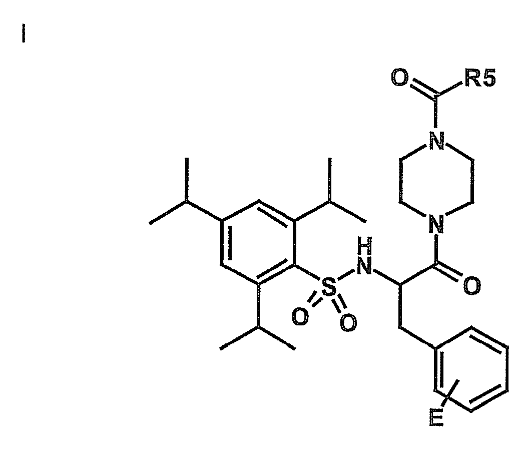 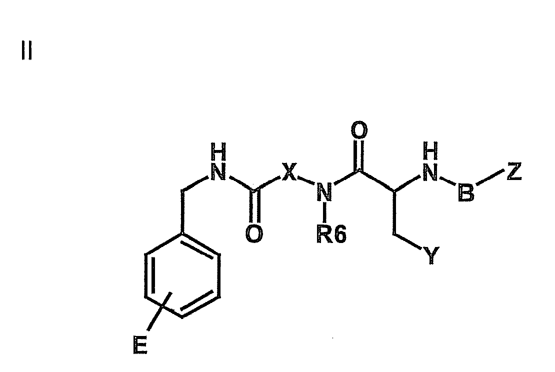 worin
E eine Gruppe aus 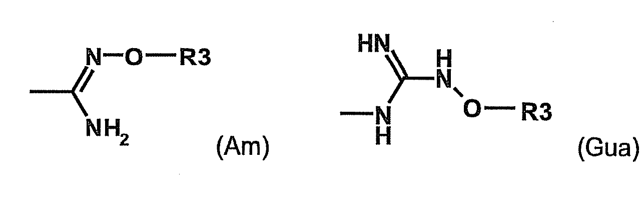 bedeutet,
B -SO₂- oder -CO- bedeutet,
X -NR¹ oder -CHR¹ bedeutet,
Z -R⁴, -OR⁴ oder -NH-R⁴ bedeutet,
Y -OR² oder -NHR² bedeutet,
R¹ jeweils unabhängig -H, -C₁-C₆-Alkyl, -C₂-C₆-Alkenyl oder -C₂-C₆-Alkinyl unsubstituiert oder substituiert bedeutet,
R² -H, -OR¹, -COR¹, -COOR¹ oder -CON(R¹)₂ bedeutet,
R³ -H, -C₁-C₆-Alkyl, -C₂-C₆-Alkenyl oder -C₂-C₆-Alkinyl, unsubstituiert oder substituiert oder -COR⁶ oder -COOR⁶ oder einen Oligo- oder Poylalkylenoxyrest mit z.B. 2-50 -C₂-C₄-Alkylenoxy, z.B. Ethylenoxyresten bedeutet,
R⁴ -H, -C₁-C₆-Alkyl, -C₂-C₆-Alkenyl oder -C₂-C₆-Alkinyl unsubstituiert oder substituiert oder einen cyclischen Rest bedeutet und
R⁵ -OR⁶, -N(R⁶)₂, -C₁-C₆-Alkyl, -C₂-C₆-Alkenyl oder -C₂-C₆-Alkinyl unsubstituiert oder substituiert bedeutet, und
R⁶ -H, -C₁-C₆-Alkyl, -C₂-C₆-Alkenyl oder -C₂-C₆-Alkinyl unsubstituiert oder substituiert oder einen cyclischen Rest bedeutet,
   wobei jeder cyclische Rest einen oder mehrere Substituenten, z.B. ausgewählt aus -C₁-C₃-Alkyl, -OR⁶ (z.B. -OH oder -C₁-C₃-Alkoxy), Halogen, =O, -NO₂ -CN, -COOR⁶, -N(R⁶)₂, -NR⁶COR⁶, -NR⁶CON(R⁶)₂ und -OCOR⁶ tragen kann,
   und wobei jedes Alkyl, Alkenyl und Alkinyl geradkettig oder verzweigt sein kann und einen oder mehrere Substituenten, z.B. ausgewählt aus Halogen (F, Cl, Br, I), -OR⁶, -OCOR⁶, -N(R⁶)₂, -NR⁶COR⁶, COOR⁶, -NR⁶COR⁶ oder einem cyclischen Rest, tragen kann
   oder Salze dieser Verbindungen sowie gegebenenfalls pharmazeutisch übliche Träger, Verdünnungs- oder/und Hilfsmittel enthält.

Vorzugsweise ist das Arzneimittel ein oral verabreichbares Mittel. Besonders bevorzugt wird das Arzneimittel zur Hemmung des Urokinase-Plasminogenaktivators eingesetzt.

Bevorzugt sind Verbindungen der allgemeinen Formel III 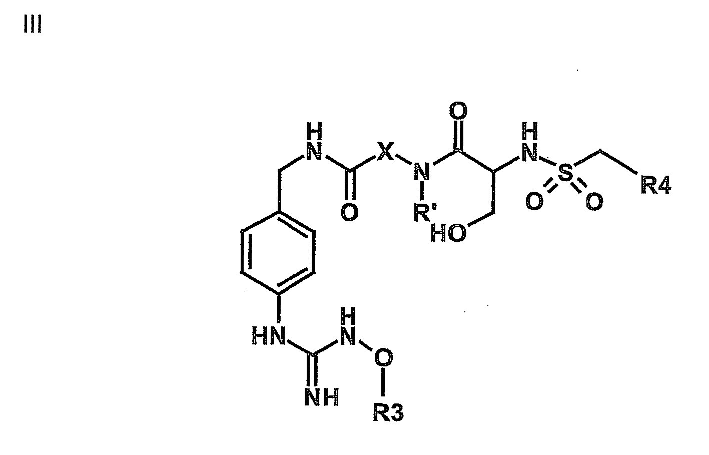 worin
X, R¹, R³,R⁴ und R⁶ wie oben definiert sind,
oder deren Salze.

Die Gruppe E befindet sich vorzugsweise in para-Position des Phenylringes bei den Verbindungen I und II. Besonders bevorzugt sind Verbindungen der allgemeinen Formel I, worin E Am ist.

Die erfindungsgemäßen Verbindungen weisen eine modifizierte Amidino-oder Guanidinofunktion E, vorzugsweise eine Hydroxyguanidino- oder Hydroxyamidinofunktion auf. Derartige Modifikationen waren lediglich als Synthesezwischenprodukte bei der Herstellung von Urokinase-Inhibitoren des Guanidino- oder Amidinotyps bekannt. Eine pharmazeutische Wirksamkeit wurde bisher nicht vermutet.

Die Verbindungen können als Salze, vorzugsweise als physiologisch verträgliche Säuresalze, z.B. Salze von Mineralsäuren, besonders bevorzugt als Hydrochloride oder Hydrogensulfate, oder als Salze von geeigneten organischen Säuren, z.B. von organischen Carbon- oder Sulfonsäuren, wie etwa Tartrate, Mesylate oder Besylate, vorliegen. Besonders bevorzugt sind die Hydrogensulfate. Die Verbindungen können als optisch reine Verbindungen oder als Gemische von Enantiomeren oder/und Diastereomeren vorliegen.

Cyclische Reste können einen oder mehrere gesättigte, ungesättigte oder aromatische Ringe enthalten. Bevorzugte Beispiele für cyclische Reste sind Cycloalkylreste, Arylreste, Heteroarylreste und bicyclische Reste. Besonders bevorzugt sind mono- oder bicyclische Reste. Die cyclischen Reste enthalten vorzugsweise 4 bis 30, insbesondere 5-10 Kohlenstoff- und Heteroatome als Ringatome, sowie gegebenenfalls einen oder mehrere Substituenten wie zuvor angegeben. Heterocyclische Systeme enthalten bevorzugt ein oder mehrere O-, S- oder/und N-Atome. Bevorzugte bicyclische Ringsysteme sind solche mit einem -CO-Rest.

Alkyl-, Alkenyl- und Alkinylgruppen enthalten vorzugsweise bis zu 4 Kohlenstoffatome. R¹ ist bevorzugt H oder ein gegebenenfalls substituierter -C₁-C₄-Alkylrest, z.B. -CH₃ oder ein C₁-C₆-Alkyl-arylrest, so dass -CO-X-NR¹ z.B. einen Glycyl-, Alanyl-, Phenylalanyl- oder Homophenylalanylrest darstellen kann. R² ist besonders bevorzugt H oder ein C₁-C₃-Alkylrest, so dass Y z.B. einen OH- oder O-C₁-C₃-Alkylrest darstellen kann. R³ ist besonders bevorzugt H. In den Verbindungen I bedeutet R⁵ bevorzugt -NHR⁶, besonders bevorzugt -NH(C₁-C₅)-Alkyl, unsubstituiert oder substituiert, z.B. -NHC₂H₅ oder -OR⁶, besonders bevorzugt -O(C₁-C₃)Alkyl, unsubstituiert oder substituiert, z.B. Ethyloxy oder Benzyloxy, oder -O-Aryl, z.B. Phenyloxy. In den Verbindungen II und III ist R⁶ vorzugsweise -H oder C₁-C₃-Alkyl.

Bevorzugt sind Verbindungen, worin das Strukturelement Z für R⁴ steht, worin R4 einen Alkylrest mit einem cyclischen Substituenten, z.B. einem gegebenenfalls substituierten Phenylrest oder einem bicyclischen Rest, wie etwa 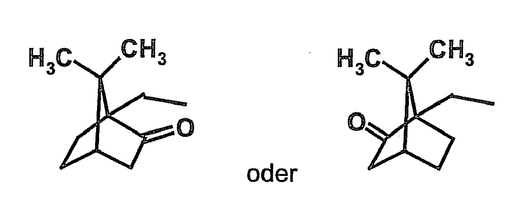 bedeutet.

Besonders bevorzugte Verbindungen sind solche, worin R⁴ einen substituierten oder unsubstituierten C₁-C₃-Alkyl-Aryl-Rest bedeutet, z.B. einen Benzylrest, der gegebenenfalls in meta- oder para-Position mit Halogen oder/und -NO₂ substituiert sein kann, wobei das Halogen ausgewählt wird aus F, Cl, Br und I, besonders bevorzugt Cl und Br.

Am meisten bevorzugt sind die Verbindungen
N-α-(2,4,6-Triisopropylphenyl-sulfonyl)-3-hydroxyamidino-(L)-phenylalanin-4-ethoxy-carbonylpiperazid (WX- 671),
N-α-(2,4,6-Triisopropyl-phenylsulfonyl)-3-hydroxyamidino-(D)-phenyl-alanin-4-ethoxycarbonyl-piperazid,
N-α-(2,4,6-Triisopropylphenylsulfonyl)-3-hydroxyamidino-(D,L)-phenylalanin-4-ethoxycarbonylpiperazid,
N-α-(2,4,6-Triisopropylphenylsulfonyl)-3-hydroxyguanidino-(L)-phenylalanin-4-ethoxy- carbonylpiperazid (WX-683),
N-α-(2,4,6-Triisopropylphenylsulfonyl)-3-hydroxyguanidino-(D)-phenylalanin-4-ethoxycarbonylpiperazid,
N-α-(2,4,6-Triisopropylphenyl-sulfonyl)-3-hydroxyguanidino-(D,L)-phenylalanin-4-ethoxy-carbonyl-piperazid,
N-α-(2,4,6-Triisopropylphenylsulfonyl)-3-hydroxy-guanidino-(L)-phenylalanin-4-ethylaminocarbonylpiperazid (WX-685),
N-α-(2,4,6-Triisopropylphenylsulfonyl)-3-hydroxyguanidino-(D)-phenylalanin-4-ethyl-aminocarbonylpiperazid,
N-α-(2,4,6-Triisopropylphenylsulfonyl)-3-hydroxyguanidino-(D,L)-phenylalanin-4-ethylaminocarbonylpiperazid,
Benzylsulfonyl-(D)-Ser-Gly-(4-hydroxyguanidinobenzyl)amid (WX-678), 4-Chlorbenzylsulfonyl-(D)-Ser-N-Me-Ala-(4-hydroxyguanidinobenzyl)amid, 4-Chlorbenzylsulfonyl-(D)-Ser-Gly-(4-hydroxyguanidinobenzyl)amid, Benzylsulfonyl-(D)-Ser-N-Me-Gly-(4-hydroxyguanidinobenzyl)amid, 4-Chlorbenzylsulfonyl-(D)-Ser-Ala-(4-hydroxyguanidinobenzyl)amid sowie deren Salze, z.B. die Hydrogensulfate, wie etwa WX-671 · HSO₄.

Die erfindungsgemäßen Verbindungen können gegebenenfalls zusammen mit geeigneten pharmazeutischen Hilfs- oder Trägerstoffen zur Herstellung von Arzneimitteln verwendet werden. Dabei ist eine Verabreichung in Kombination mit anderen Wirkstoffen, z.B. anderen Urokinase-Inhibitoren, wie etwa Antikörpern und/oder Peptiden, aber auch mit Chemotherapeutika und Zytostatika oder/und zytostatischen Wirkstoffen möglich.

Die Arzneimittel können bei Mensch und Tieren topisch, rektal oder parenteral, z.B. intravenös, subkutan, intramuskulär, intraperitoneal, sublingual, nasal oder/und inhalativ, z.B. in Form von Tabletten, Dragees, Kapseln, Pellets, Suppositorien, Lösungen, Emulsionen, Suspensionen, Liposomen, Inhalationssprays oder transdermalen Systemen, wie Pflastern, und besonders bevorzugt oral , z.B. als Slow-Release/Retard-Formulierung verabreicht werden.

Die erfindungsgemäßen Verbindungen sind zur Bekämpfung von Krankheiten geeignet, die mit einer pathologischen Überexpression von uPA oder/und Urokinase-Plasminogen-Aktivatorrezeptor (uPAR) assoziiert sind. Sie sind beispielsweise in der Lage, hocheffizient das Wachstum oder/und die Ausbreitung maligner Tumoren sowie die Metastasierung von Tumoren zu hemmen. Beispiele hierfür sind Tumorerkrankungen, z.B. Brustkrebs, Lungenkrebs, Blasenkrebs, Magenkrebs, Cervixkrebs, Ovarienkrebs, Nierenkrebs, Prostatakrebs und Weichgewebesarkome, insbesondere mit einer hohen Metastasierungsrate assoziierte Tumore. Dabei können die Verbindungen gegebenenfalls zusammen mit anderen Tumormitteln oder mit anderen Behandlungsarten, z.B. Bestrahlung oder/und chirurgischen Eingriffen, eingesetzt werden.

Weiterhin sind die erfindungsgemäßen Verbindungen auch für andere uPAassoziierte oder/und uPAR assoziierte Erkrankungen wirksam. Beispiele für derartige Erkrankungen sind etwa pulmonärer Bluthochdruck und/oder Herzerkrankungen (z.B. WO 02/00248), Magen- und Darmerkrankungen, wie etwa inflammatorische Darmerkrankung, prämaligne Colonadenome, entzündliche Erkrankungen, wie etwa septische Arthritis, Osteoarthritis, rheumatoide Arthritis, oder andere Erkrankungen, wie Osteoporose, Cholesteatomie, Haut- und Augenerkrankungen sowie virale oder bakterielle Infektionen, wobei ausdrücklich auf die in EP-A-0 691 350, EP-A-1 182 207 und US-Patent 5,712,291 genannten Erkrankungen Bezug genommen wird.

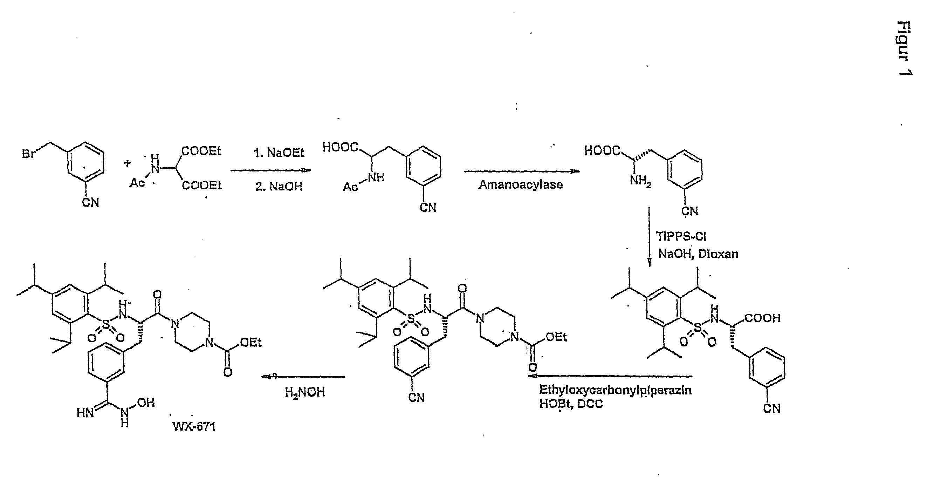

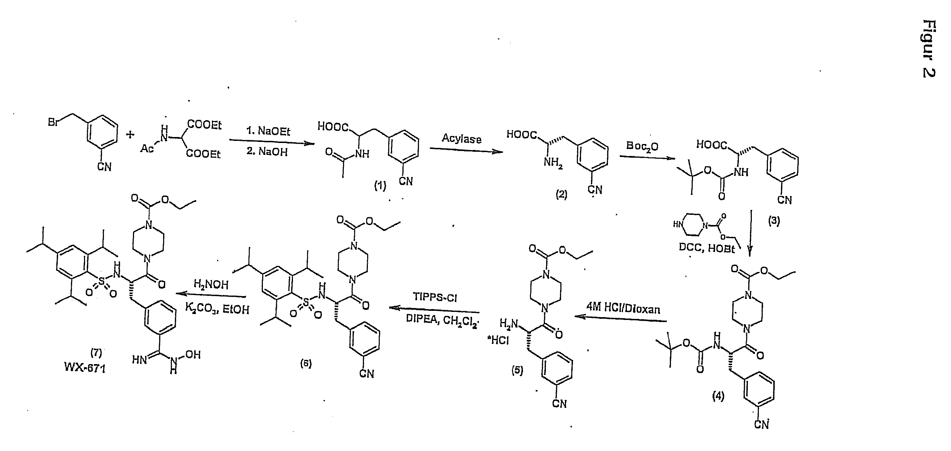

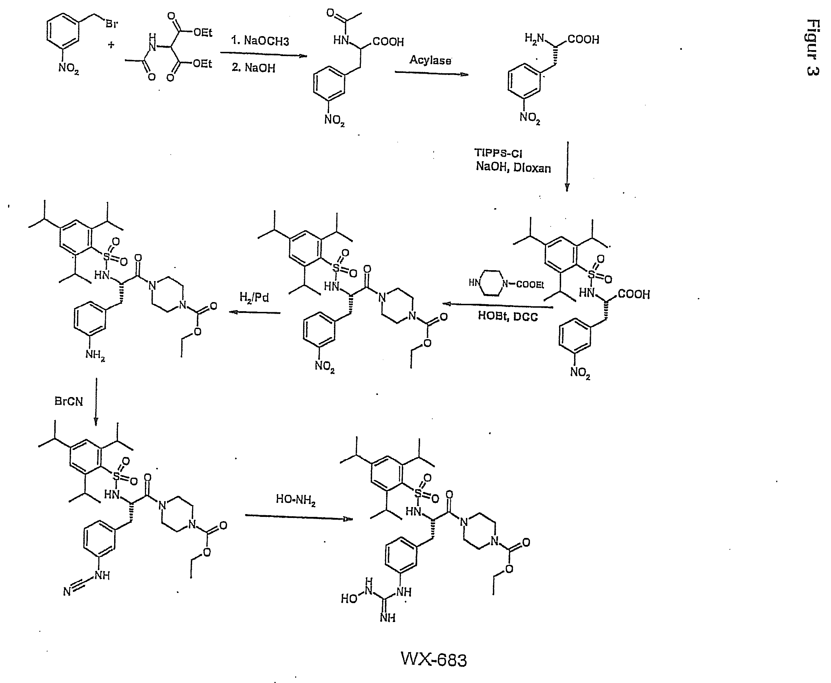

Die Verbindungen der allgemeinen Formel I können beispielsweise wie in den Syntheseschemata in Figur 1, 2 und 3 hergestellt werden.

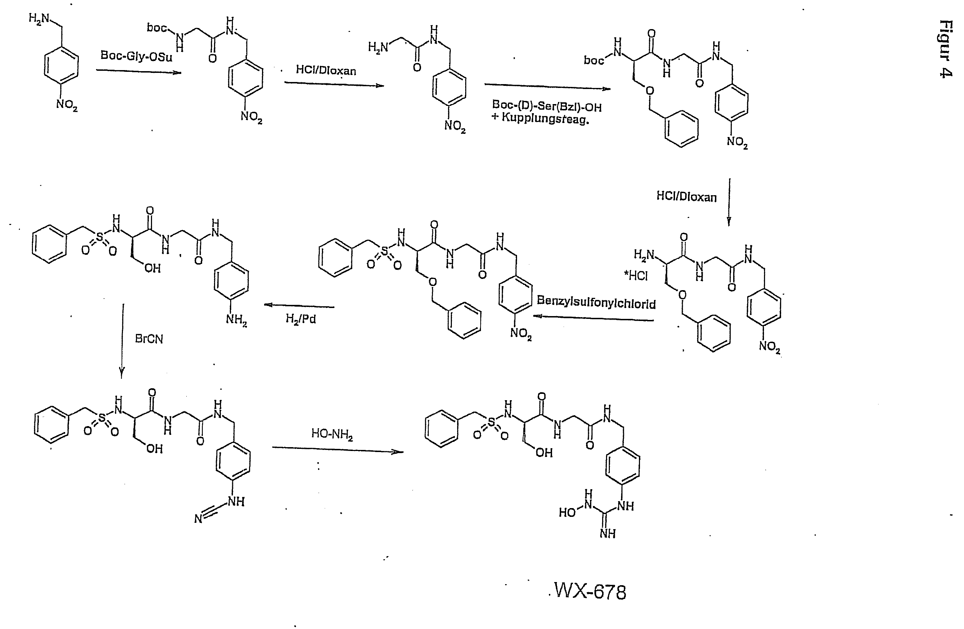

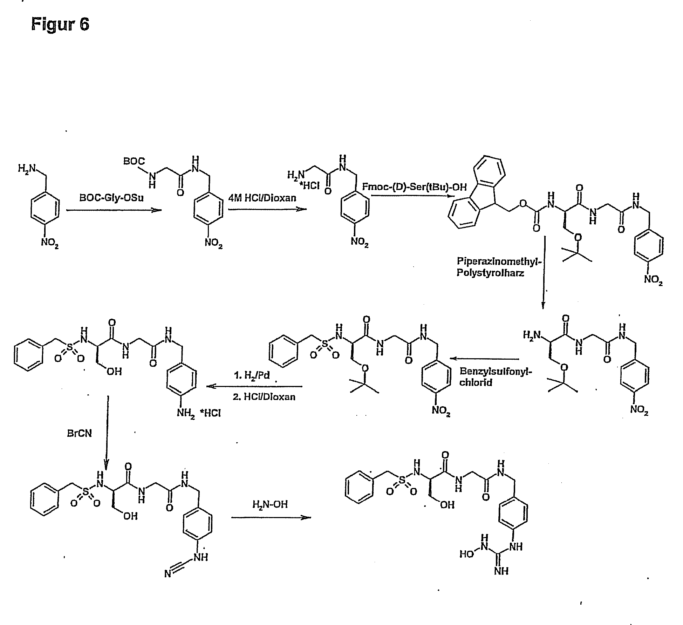

Die Verbindungen der allgemeinen Formel II und III können beispielsweise wie in den Syntheseschemata in Figur 4 und 6 hergestellt werden.

Erfindungsgemäße uPA-Inhibitoren sind vorzugsweise dadurch gekennzeichnet, dass sie mindestens eine 5-fache, vorzugsweise eine 10-fache und besonders bevorzugt eine 100-fache höhere Bioverfügbarkeit aufweisen als die entsprechenden Urokinase-Inhibitoren dieser Klasse mit einer nicht modifizierten Amidino- oder Guanidinofunktion nach oraler Verabreichung.

Überraschenderweise wurde festgestellt, dass die erfindungsgemäßen uPA-Inhibitoren nicht nur eine verbesserte Bioverfügbarkeit aufweisen, sondern auch eine deutlich verbesserte Aktivität gegenüber einem Primärtumor aufweisen.

Die erfindungsgemäßen Substanzen der Formel I, II und III können alleine oder in Kombination mit anderen physiologisch wirksamen Substanzen eingesetzt werden, z.B. mit Radiotherapeutika oder mit zytotoxischen oder/und zytostatischen Mitteln, z.B. Chemotherapeutika, wie etwa cis-Platin, Doxorubicin, 5-Fluoruracil, Taxolderivaten, oder/und sonstigen chemotherapeutischen Agenzien, beispielsweise ausgewählt aus der Gruppe der alkylierenden Agenzien, Antimetaboliten, Antibiotika, Epidophyllotoxine und Vinca-Alkaloide. Ebenfalls möglich ist eine Kombination mit Strahlentherapie oder/und chirurgischen Eingriffen.

Durch die Erfindung wird ein Verfahren zur Urokinasehemmung bei Lebewesen, insbesondere dem Menschen, durch Verabreichung einer wirksamen Menge mindestens einer Verbindung der allgemeinen Formel I, II oder/und III bereitgestellt. Die zu verabreichende Dosis hängt ab von der Art und Schwere der zu behandelnden Erkrankungen. Beispielsweise liegt die Tagesdosis im Bereich von 0,01-100 mg/kg Wirksubstanz.

Schließlich betrifft die Erfindung neue Inhibitoren des Urokinase-Plasminogen-Aktivators der allgemeinen Formel I, II und III.

Die Erfindung soll durch die folgenden Figuren und die Beispiele näher erläutert werden.

**Die** **Figuren 1-4** **und** **6** zeigen schematisch die Herstellung der Verbindungen WX-671 (Figuren 1 und 2), WX-683 (Figur 3) und WX-678 (Figuren 4 und 6).

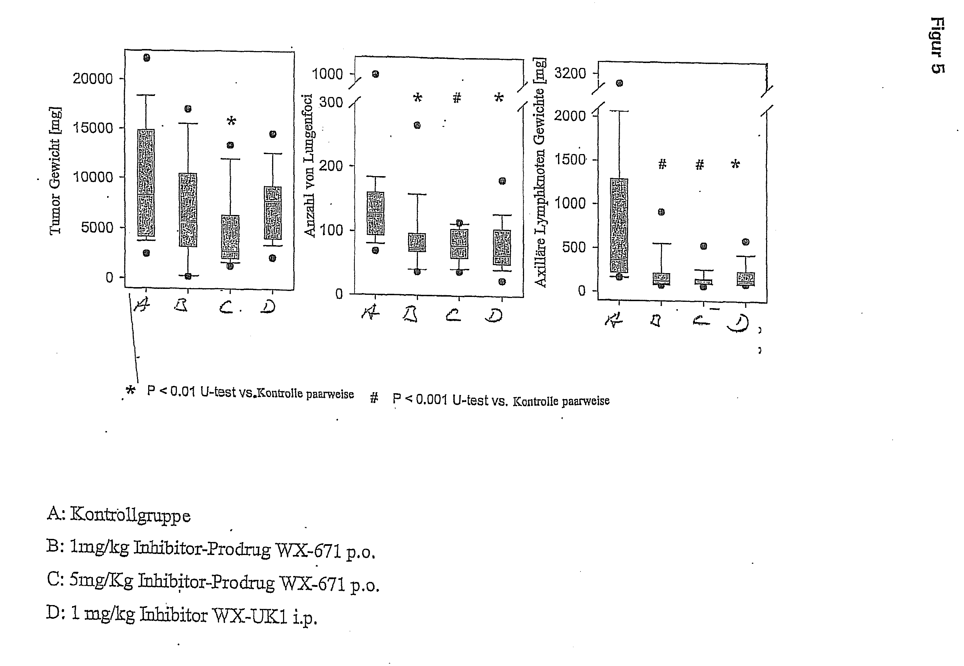

**Figur 5** zeigt Ergebnisse im Brustkrebs-Modell der Ratte mit der erfindungsgemäßen Substanz WX-671 im Vergleich zu Kontrollen.

### Beispiele

### Beispiel 1: Herstellung von WX-671

### 1.1 N-Acetyl-3-Cyano-(D/L)-Phenylalanin

3-Cyanobenzylbromid (935 g; 4,77 mol), Acetamidomalonsäurediethylester (1036 g; 4,77 mol) und Kaliumiodid (20 g) wurden bei 98 °C unter Argon in 5 I Dioxan gelöst und 5 h gerührt. Anschließend wurde eine Lösung von Natriumethanolat (340 g; 5 mmol) in 2 I Ethanol während 3 h zugetropft. Danach wurden 4,4 I 3 M NaOH zugegeben und weitere 2 h bei 98 °C und dann über Nacht bei Raumtemperatur (RT) gerührt. Die Lösung wurde im Vakuum auf -2 I eingeengt, 3 I destilliertes Wasser zugegeben und auf RT abgekühlt. Nach Einstellen des pH Wertes auf >9 wurde 3x mit 1 I Ethylacetat extrahiert. Die wässrige Phase wurde mit 4M HCl auf pH 1 gebracht (ca. 4 I 4M HCl) und 4x mit 1,2 I Ethylacetat extrahiert. Die vereinigten organischen Phasen wurden mit gesättigtem NaCl gewaschen, das Lösungsmittel abgezogen und aus Ethylacetat umkristallisiert. Ausbeute 815 g (3,5 mol) 73 %

### 1.2 3-Cyano-(L)-Phenylalanin (Racematspaltung)

N-Acetyl-3-Cyano-(D/L)-Phenylalanin (696 g; 3 mol) wurde in 2 I Wasser und 3 I 1M NaOH gelöst, der pH Wert mit ca. 10 ml 4M HCl auf 7,2 eingestellt und auf 37 °C temperiert. Nach Zugabe von 28 g Acylase I (Aspergillus Melleus) wurde 60 h bei 37 °C langsam gerührt. Nach Filtrieren des entstandenen Niederschlages (Produkt) wurde die Lösung auf ca. 1,5 I Volumen eingeengt und der Niederschlag abfiltriert. Die vereinten Filterkuchen wurden in 0,5 I Wasser suspendiert, gerührt, erneut filtriert und im Vakuum getrocknet.
Ausbeute 190 g (33 %), Reinheit 99 % (HPLC)

### 1.3 Triisopropylphenylsulfonyl (TIPPS)-3-Cyano-(L)- Phenylalanin

3-Cyano-(L)-Phenylalanin (133 g, 700 mmol) wurde in 1,2 I Dioxan und 1540 ml 1M NaOH gelöst und auf 5°C gekühlt. Triisopropylphenylsulfonylchlorid (TIPPS-Cl) (212 g; 700 mmol) wurde in 1 I Dioxan gelöst und über 1 Std. zugetropft. Anschließend wurde weiteres TIPPS-Cl und NaOH zugesetzt und gerührt bis kein Edukt mehr zu detektieren war. Die orangefarbene Lösung wurde mit 4M HCl auf pH 5 angesäuert und 2x mit MTBE extrahiert. Die vereinten organischen Lösungen wurden 2x mit NaCl-Lösung extrahiert und das Lösungsmittel anschließend im Vakuum abgezogen und mit Toluol nachrotiert.
Ausbeute 302 g (88 %) mit 93 % Reinheit (HPLC)

### 1.4 TIPPS-3-Cyano-(L)-Phenylalanin-4-Ethoxycarbonylpiperazid

TIPPS-3-Cyano-(L)-Phenylalanin (215 g; 0,435 mmol; 93 % Reinheit), Ethyloxycarbonylpiperazin (68,8 g; 0,435 mmol) und 1-Hydroxybenzotriazol (13,3 g; 0,087 mmol) wurden in 650 ml DMF gelöst und auf 10 °C gekühlt. Während 2 h wurde eine Lösung von Dicyclohexylcarbodiimid (98,7 g; 0,478 mmol) in 216 ml DMF zugetropft und die Reaktionslösung über Nacht bei RT gerührt. Nach Abziehen des Lösungsmittels wurde der Rückstand in 436 ml MTBE gelöst, der Niederschlag abfiltriert, und die organische Lösung je 2x mit 5 % KHSO₄, 5 % NaHCO₃ und destilliertem Wasser extrahiert. Das Lösungsmittel wurde im Vakuum abgezogen, mit Toluol nachrotiert und das Produkt im Vakuum getrocknet.
Ausbeute 261 g hellgelber Feststoff (90 %) mit 90 % Reinheit (HPLC)

### 1.5 TIPPS-3-Hydroxyamidino-(L)-Phenylalanin-4-Ethoxycarbonylpiperazid (WX-671)

TIPPS-3-Cyano-(L)-Phenylalanin-4-Ethoxycarbonylpiperazid (130 g; 196 mmol; 90 % Reinheit), Hydroxylamin Hydrochlorid (22 g; 313 mmol) und Triethylamin (63 g, 626 mmol) wurden in 470 ml Ethanol gelöst und 1 Tag bei RT gerührt. Nach Abziehen des Lösungsmittels wurde der Rückstand in 300 ml Ethylacetat aufgenommen und je 2x mit mit 5 % KHSO₄, 5 % NaHCO₃ und destilliertem Wasser extrahiert. Nach Abziehen des Lösungsmittels wurde das Rohprodukt im Vakuum getrocknet und anschließend aus Ethylacetat/Ether umkristallisiert.
Ausbeute 63 g (50 %) weißes Pulver mit 97 % Reinheit (HPLC)

### Beispiel 2: Herstellung von WX-678

### 2.1 H-Gly-4-Nitrobenzylamid Hydrochlorid

4-Nitrobenzylamin Hydrochlorid (1 g; 5,3 mmol) und Diisopropylethylamin (1,8 ml; 10,6 mmol) wurden bei RT in 70 ml Dichlormethan gelöst. BOC-Gly-OSu (1,44 g; 5,3 mmol) wurde zugegeben und die Lösung über Nacht bei RT gerührt. Nach Abziehen des Lösungsmittels am Rotationsverdampfer wurde der Rückstand in 50 ml Ethylacetat aufgenommen und je 2x mit 5 % KHSO₄, 5 % NaHCO₃ und destilliertem Wasser extrahiert. Die organische Phase wurde über Na₂SO₄ getrocknet, das Lösungsmittel abgezogen und mit Toluol nachrotiert. Das entstandene Öl wurde ohne weitere Aufarbeitung direkt in 15 ml 4M HCl/Dioxan gelöst und bei RT gerührt. Nach kurzer Zeit beginnt das Produkt zu prezipitieren. Nach 1 h wurde das Lösungsmittel abgezogen, der Feststoff in 100 ml Ethylacetat suspendiert, abfiltriert und mit Petrolether gewaschen. Der weiße Feststoff wurde im Vakuum getrocknet.
Ausbeute: 1,17 g (90 %)

### 2.2 Fmoc-(D)-Ser(tBu)-Gly-4-Nitrobenzylamid

H-Gly-4-Nitrobenzylamid Hydrochlorid (1,17 g; 4,77 mmol), Fmoc-(D)-Ser(tBu)-OH (1,83 g; 4,77 mmol) und Diisopropylethylamin (2,5 ml; 14,3 mmol) wurden in 40 ml DMF:Dichlormethan 1:1 gelöst. Nach Zugabe von PyBOP (2,73 g; 5,25 mmol) wurde die Lösung bei RT gerührt. Nach 2,5 h wurde das Lösungsmittel im Hochvakuum komplett abgezogen und der Rückstand in 300 ml Dichlormethan gelöst und 2x mit TIPPS-3-Amino-(L)-Phenylalanin-4-Ethoxycarbonylpiperazid und 1x mit konz. NaCl extrahiert. Die organische Phase wurde über Na₂SO₄ getrocknet, das Lösungsmittel abgezogen und mit Toluol nachrotiert. Das Produkt wurde im Hochvakuum getrocknet.

### 2.3 H-(D)-Ser(tBu)-Gly-4-Nitrobenzylamid Hydrochlorid

Fmoc-(D)-Ser(tBu)-Gly-4-Nitrobenzylamid (3,1 g) wurde in 100 ml Dichlormethan suspendiert und 5 g Piperazinomethyl-Polystyrolharz (= 5,5 mmol Piperazin) wurden zugegeben. Nach 1 h trat noch keine Reaktion ein und es wurden 5 mmol Diisopropylethylamin (856 µl) zugegeben. Nach einem Tag trat immer noch keine keine Reaktion ein und es wurde erneut Diisopropylethylamin (5 mmol; 856 µl) zugegeben und die Suspension am Rotationsverdampfer auf ca. 20 % des Ursprungsvolumens eingeengt. Nach 5 Tagen war ca. 50 % Produkt enstanden, es wurde erneut Diisopropylethylamin (5 mmol; 856 µl) zugegeben und die Lösung mit 20 ml DMF versetzt, um die Löslichkeit zu steigern. Nach weiteren 6 Tagen wurde das Harz abfiltriert und das Lösungsmittel abgezogen. Das verbliebene Öl wurde im Ultraschallbad mit Petrolether behandelt und abdekantiert. Der Vorgang wurde mit Diethylether wiederholt um das Nebenprodukt Dibenzofulven abzutrennen. Anschließend wurde das Öl in 30 ml Dichlormethan gelöst und das Produkt mit einer Lösung aus 2 ml 4M HCl/Dioxan in 20 ml Dichlormethan als Hydrochlorid gefällt. Die Fällung wurde mit 50 ml Petrolether vervollständigt, die überstehende Lösung abdekantiert und der Niederschlag im Vakuum getrocknet.
Ausbeute 1,68 g hellgelbes Pulver (90 % für die beiden letzten Synthesestufen)

### 2.4 Benzylsulfony-(D)-Ser(tBu)-Gly-4-Nitrobenzylamid

1,68 g H-(D)-Ser(tBu)-Gly-4-Nitrobenzylamid Hydrochlorid (4,32 mmol) und Diisopropylethylamin (2,22 ml; 12,96 mmol) wurden in 80 ml Dichlormethan gelöst. Nach Zugabe von Benzylsulfonylchlorid (824 mg; 4,32 mmol) wurde bei RT gerührt. Nach 3,5 h wurde erneut Benzylsulfonylchlorid (100 mg) und Diisopropylethylamin (500 µl) zugegeben, um die Reaktion zu vervollständigen. Nach weiteren 2 h wurde das Lösungsmittel abgezogen, der Rückstand in 130 ml Ethylacetat aufgenommen, die Lösung je 2x mit 5 % NaHCO₃ und 5 % KHSO₄ und 1x mit konzentrietem NaCl extrahiert. Die organische Phase wurde über Na₂SO₄ getrocknet, das Lösungsmittel abgezogen und mit Toluol nachrotiert. Das Produkt wurde im Hochvakuum getrocknet.
Ausbeute 1,85 g hellgelbes Pulver (84 %)

### 2.5 Benzylsulfony-(D)-Ser(tBu)-Gly-4-Aminobenzylamid

1,85 g Benzylsulfony-(D)-Ser(tBu)-Gly-4-Nitrobenzylamid (3,65 mmol) wurden in 50 ml Methanol gelöst und über Pd/C hydriert. Nach 8 h wurde der Katalysator abfiltriert, das Lösungsmittel abgezogen, mit Toluol nachrotiert und das Rohprodukt in wenig Dichlormethan gelöst. Beim Abziehen des Lösungsmittels am Rotationsverdampfer schäumte das Produkt auf und erstarrte im Vakuum.
Ausbeute 1,6 g hellgelbes Pulver (92 %)

### 2.6 Benzylsulfony-(D)-Ser-Gly-4-Aminobenzylamid Hydrochlorid

Zum Abspalten der tert-Butyl-Schzutzgruppe wurde Benzylsulfony-(D)-Ser(tBu)-Gly-4-Aminobenzylamid (1 g) in 20 ml 4M HCl/Dioxan suspendiert und bei RT gerührt. Nach 7 h wurde das Lösungsmittel im Vakuum abgezogen, mit Toluol nachrotiert und das Produkt im Vakuum getrocknet.
Ausbeute 1,07 g hochreines Produkt (quantitativ)

### 2.7 Benzylsulfony-(D)-Ser-Gly-4-Cyanoaminobenzylamid

Benzylsulfony-(D)-Ser-Gly-4-Aminobenzylamid Hydrochlorid (500 mg; 1,09 mmol), Natriumacetat (224 mg; 2,725 mmol) und Bromcyan (127 mg; 1,2 mmol) wurden in absolutem, über Molekularsieb getrocknetem Ethanol gelöst und 3 h bei RT gerührt. Die Lösung wurde im Eisbad gekühlt und die ausgefallenen Salze abfiltriert. Die Lösung wurde direkt im nächsten Reaktionsschritt eingesetzt.

### 2.8 Benzylsulfony-(D)-Ser-Gly-4-Hydroxyguanidinobenzylamid

Zur ethanolischen Rohproduktlösung von Benzylsulfony-(D)-Ser-Gly-4-Cyanoaminobenzylamid wurde Hydroxylamin Hydrochlorid (83,4 mg; 1,2 mmol) und Diisopropylethylamin (195 µl; 1,2 mmol) gegeben und die Reaktionsmischung über Nacht bei 0 °C gerührt. Die ausgefallenen Salze wurden abfiltriert und das Lösungsmittel abgezogen. Das Produkt wurde über prep. reversed Phase HPLC gereinigt.
Ausbeute 120 mg (0,25 mmol; 21 %); Reinheit (HPLC): 95 %; ESI-MS: m/z = 479,0 (M+H⁺); berechnet für C₂₀H₂₆N₆O₆S₁: 478

### Beispiel 3: Hergestellung von WX-683

### 3.1 N-Acetyl-3-Nitro-(D/L)-Phenylalanin

3-Nitrobenzylbromid (1000 g; 4,63 mol), Acetamidomalonsäurediethylester (1005 g; 4,63 mol) und Kaliumiodid (20 g) wurden bei 98 °C unter Argon in 4 I Dioxan gelöst und 5 h gerührt. Anschließend wurde eine Lösung von Natriumethanolat (340 g; 5 mmol) in 2 I Ethanol während 3 h zugetropft. Danach wurden 550 g NaOH (13,75 mol) zugegeben und weitere 2 h bei 98 °C und dann über Nacht bei RT gerührt. Die Lösung wurde im Vakuum auf ∼2 I eingeengt, 3 I destilliertes Wasser zugegeben und auf RT abgekühlt. Nach Einstellen des pH Wertes auf >9 wurde 3x mit 1 I Ethylacetat extrahiert. Die wäßrige Phase wurde mit 4M HCl auf pH 1 gebracht (ca. 4I 4M HCl) und 4x mit 1,2 I Ethylacetat extrahiert. Die vereinigten organischen Phasen wurden mit gesättigtem NaCl gewaschen, das Lösungsmittel abgezogen und aus Ethylacetat umkristallisiert.
Ausbeute 1011 g (3,2 mol) 69 %

### 3.2 3-Nitro-(L)-Phenylalanin (Racematspaltung)

N-Acetyl-3-Nitro-(D/L)-Phenylalanin (1000 g; 3,17 mol) wurde in 2 I Wasser und 3 I 1M NaOH gelöst, der pH Wert mit ca. 10 ml 4M HCl auf 7,2 eingestellt und auf 37 °C temperiert. Nach Zugabe von 28 g Acylase I (Aspergillus Melleus) wurde 60 h bei 37 °C langsam gerührt. Nach Filtrieren des entstandenen Niederschlages (Produkt) wurde die Lösung auf ca. 1,5 I Volumen eingeengt und der Niederschlag abfiltriert. Die vereinten Filterkuchen wurden in 0,5 I Wasser suspendiert, gerührt, erneut filtriert und im Vakuum getrocknet.
Ausbeute 245 g (37 %), Reinheit 99 % (HPLC)

### 3.3 TIPPS-3-Nitro-(L)-Phenylalanin

3-Nitro-(L)-Phenylalanin (210 g, 1 mol) wurde in 1,2 I Dioxan und 500 ml 4M NaOH gelöst und auf 5 °C gekühlt. TIPPS-Cl (363 g; 1,2 mol) wurde in 1 I Dioxan gelöst und über 1 h zugetropft. Anschließend wurde weiteres TIPPS-Cl und NaOH zugesetzt und gerührt bis kein Edukt mehr zu detektieren war. Die orangefarbene Lösung wurde mit 4M HCl auf pH 5 angesäuert und 2x mit MTBE extrahiert. Die vereinten organischen Lösungen wurden 2x mit NaCl-Lösung extrahiert und das Lösungsmittel anschließend im Vakuum abgezogen und mit Toluol nachrotiert.
Ausbeute 427 g (68 %) mit 76 % Reinheit (HPLC)

### 3.4 TIPPS-3-Nitro-(L)-Phenylalanin-4-Ethoxycarbonylpiperazid

TIPPS-3-Nitro-(L)-Phenylalanin (210 g; 0,44 mmol; 76 % Reinheit), Ethyloxycarbonylpiperazin (69,7 g; 0,44 mmol) und 1-Hydroxybenzotriazol (101 g; 660 mmol) wurden in 650 ml DMF gelöst und auf 10 °C gekühlt. Während 2 h wurde eine Lösung von Dicyclohexylcarbodiimid (100 g; 0,484 mmol) in 216 ml DMF zugetropft und die Reaktionslösung über Nacht bei RT gerührt. Nach Abziehen des Lösungsmittels wurde der Rückstand in 436 ml MTBE gelöst, der Niederschlag abfiltriert, und die organische Lösung je 2x mit 5 % KHSO₄, 5 % NaHCO₃ und destilliertem Wasser extrahiert. Das Lösungsmittel wurde im Vakuum abgezogen, mit Toluol nachrotiert und das Produkt im Vakuum getrocknet.
Ausbeute 278 g braunes Harz (70 %) mit 69 % Reinheit (HPLC)

### 3.5 TIPPS-3-Amino-(L)-Phenylalanin-4-Ethoxycarbonylpiperazid

TIPPS-3-Nitro-(L)-Phenylalanin-4-Ethoxycarbonylpiperazid (157 g; 176 mmol) wurde in 800 ml Ethanol gelöst, mit 19,7 g 10 % Palladium auf Aktivkohle Katalysator versetzt und 3 Tage unter langsamem Durchleiten von Wasserstoff hydriert. Nach Abfiltrieren des Katalysators wurde das Lösungsmittel im Vakuum abgezogen und das Rohprodukt über Kieselgel chromatographisch gereinigt.
Ausbeute 21 g (38 %) mit 95 % Reinheit (HPLC)

### 3.6 TIPPS-3-Cyanamido-(L)-Phenylalanin-4-Ethoxycarbonylpiperazid

TIPPS-3-Amino-(L)-Phenylalanin-4-Ethoxycarbonylpiperazid (14,6 g; 24,9 mmol), Natriumacetat (wasserfrei) (5,11 g; 62,2 mmol) und Bromcyan (2,9 g; 27,4 mmol) wurden in Ethanol gelöst und die Lösung 10 h bei RT gerührt. Nach Abziehen des Lösungsmittel wurde der Rückstand in Ethylacetat aufgenommen und die Lösung mit 5 % KHSO₄, 5 % NaHCO₃ und destilliertem Wasser extrahiert. Nach Abziehen des Lösungsmittels wurde das Rohprodukt chromatographisch über Kieselgel gereinigt.
Ausbeute 10 g (60 %) mit 92 % Reinheit

### 3.7 TIPPS-3-Hydroxyguanidino-(L)-Phenylalanin-4-Ethoxycarbonylpiperazid (WX-683)

TIPPS-3-Cyanamido-(L)-Phenylalanin-4-Ethoxycarbonylpiperazid (9,3 g; 15 mmol), Hydroxylamin Hydrochlorid (1,15 g; 16,5 mmol) und Diisopropylethylamin (3,87 g; 30 mmol) wurden in Ethanol gelöst und die Lösung bei RT über Nacht gerührt. Nach Abziehen des Lösungsmittels wurde das Produkt chromatographisch über Kieselgel gereinigt.
Ausbeute 3,87 g (39 %) Reinheit 98 % (HPLC)

### Beispiel 4: In vivo Test des uPA-Inhibitor-Prodrugs WX-671 auf Tumorausbreitung, Tumorwachstum und Metastasierung in der Ratte

### Brustkrebs-Modell

10-25 mm³ große Fragmente des BN472 Brustkrebs (Kort et al., J. Natl. Cancer Inst 72, 709-713, 1984) aus einem Spendertier wurden unter das Fettpolster einer Brustdrüse von Gruppen (n=15 pro Gruppe) 7-8 Wochen alter weiblicher Brown Norwegian Ratten implantiert. Die Behandlungen begannen 72 h nach der Tumor-Implantation und wurden täglich bis zum Töten der Tiere nach 30 Tagen wiederholt. Die Kontrollgruppe (A) erhielt 0,75 ml der substanzfreien Substanzträgerlösung, bestehend aus 5 % Ethanol, 5 % D-Mannitol und 5 % Tween 20 in Wasser, oral per Schlundsonde. Die Behandlungsgruppen (B und C) erhielten oral per Schlundsonde in einem Volumen von 0,75 ml entweder 1 mg/kg (Gruppe B) oder 5 mg/kg (Gruppe C) von WX-671 in Substanzträgerlösung. Die Vergleichsgruppe D erhielt 1 mg/kg WX-UK1 gelöst in 5 % D-Mannitol per intraperitonealer Injektion.

Das Wachstum der inokulierten Tumore wurde zweimal wöchentlich mit einer Schieblehre in den Dimensionen Länge und Breite bestimmt. Nach dem Töten der Tiere wurden die Therapieendpunkte, Tumorgewicht, Gewichte der axillären und intraperitonealen Lymphknoten sowie die Anzahl makroskopischer Lungenmetastasen bestimmt.

### Zusammenfassung der Ergebnisse

In allen Versuchen wurde durch die Behandlung mit WX-671 eine erhebliche Reduktion der Tumorgröße bzw. des Tumorgewichts und der Anzahl bzw. Masse von Tochtergeschwülsten im Vergleich zu der Kontrollgruppe erreicht. Im Mammatumor-Modell waren in der mit WX-671 behandelten Gruppe die mittleren Tumorgewichte am Ende der Behandlung um über 66 % (p.o.) im Vergleich zu der Kontrolle reduziert, während mit der Inhibitor-Vergleichssubstanz WX-UK1 i.p.-Behandlung nur eine Reduktion um ca. 5% erzielt wurde. Die Anzahl der Lungenfoci in Inhibitor-Prodrug behandelten Gruppen waren um über 42 % (p.o.) und die mittleren Gewichte der axillären Lymphknoten über 63 % (p.o.) reduziert (Figur 5).

Die Entwicklung der Körpergewichtszunahme und der Vergleich der Organgewichte zwischen Inhibitor- und Vehikel-behandelten Gruppen ließen keine Hinweise für eine etwaige erhebliche Toxizität des Inhibitors unter den beschriebenen Bedingungen erkennen.

### Beispiel 5: Herstellung und Charakterisierung von WX-671 Hydrogensulfat

### Herstellung

6,0 g der freien Base WX-671 wurden in 50 ml Aceton gelöst. 1,25 Mol-Äquivalente H₂SO₄ wurden unverdünnt zugegeben. Die Mischung wurde bei Raumtemperatur für 2 h gerührt. Das Hydrogensulfat kristallisierte aus der Lösung aus. Nach Entferung des Lösungsmittels wurde der verbleibende weiße Feststoff unter Vakuum getrocknet.

### Charakterisierung

Die Wasserlöslichkeit des Hydrogensulfats bei 25 °C war 1172,5 mg/l (berechnet als Base). Die Reinheit war ≥98 % (Flächen% nach HPLC).

5 g Hydrogensulfat wurden in 25 ml Wasser/Aceton (80/20) für 7 Tage gerührt, filtriert und für 60 h bei Raumtemperatur getrocknet. Die gemessene Stöchiometrie zeigte, dass das Salz gegenüber einer Dissoziation stabil war.

Nach dem Rühren wurde keine Zunahme des Gehalts an organischen Verunreinigungen (Bestimmung durch HPLC) gefunden.

Nach Lagerung für 1 Woche bei 90 °C als Festsubstanz wurden <1,5 % organische Verunreinigungen (Bestimmung durch HPLC) gefunden.

Auf Basis der obigen Ergebnisse eignet sich das Hydrogensulfat hervorragend zur Herstellung pharmazeutischer Zubereitungen.

Gegenstand der vorliegenden Erfindung sind ferner die folgenden Gegenstände:
1. Arzneimittel, das als Wirkstoff eine oder mehrere Verbindungen der allgemeinen Formel I und/oder II 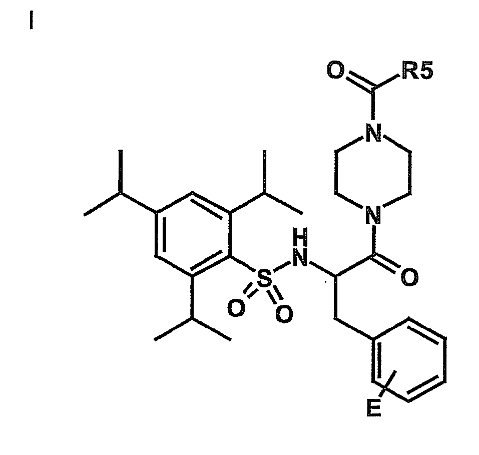 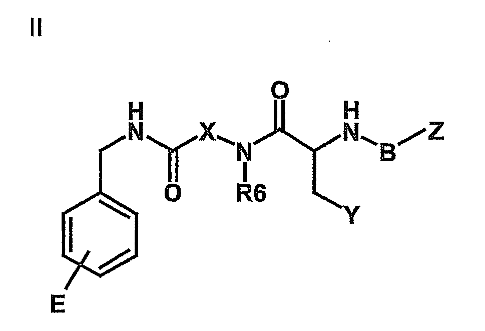 worin
   E eine Gruppe aus 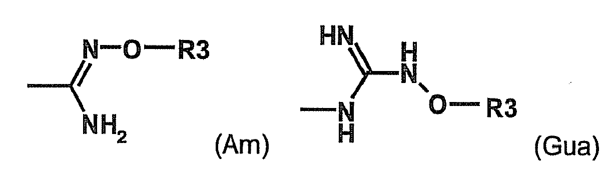 bedeutet,
   B -SO₂- oder -CO- bedeutet,
   X -NR¹ oder -CHR¹ bedeutet,
   Z -R⁴, -OR⁴ oder -NH-R⁴ bedeutet,
   Y -OR² oder -NHR² bedeutet,
   R¹ jeweils unabhängig -H, -C₁-C₆-Alkyl, -C₂-C₆-Alkenyl oder -C₂-C₆-Alkinyl unsubstituiert oder substituiert bedeutet,
   R² -H, -OR¹, -COR¹, -CON(R¹)₂,oder -COOR¹ bedeutet,
   R³ -H, -C₁-C₆-Alkyl, -C₂-C₆-Alkenyl oder -C₂-C₆-Alkinyl unsubstituiert oder substituiert oder -COR⁶ oder -COOR⁶ oder einen Oligo-oder Poylalkylenoxyrest bedeutet,
   R⁴ -H, -C₁-C₆-Alkyl, -C₂-C₆-Alkenyl oder -C₂-C₆-Alkinyl unsubstituiert oder substituiert oder einen cyclischen Rest bedeutet,
   R⁵ -OR⁶, -N(R⁶)₂, -C₁-C₆-Alkyl, -C₂-C₆-Alkenyl oder -C₂-C₆-Alkinyl unsubstituiert oder substituiert bedeutet und
   R⁶ -H, -C₁-C₆-Alkyl, -C₂-C₆-Alkenyl oder -C₂-C₆-Alkinyl unsubstituiert oder substituiert oder einen cyclischen Rest bedeutet,
   wobei jeder cyclische Rest einen oder mehrere Substituenten, ausgewählt aus -C₁-C₃-Alkyl, -C₁-C₃-Alkoxy), Halogen, -OR⁶, =O, -NO₂ -CN, -COOR⁶, -N(R⁶)₂, -NR⁶COR⁶, -NR⁶CON(R⁶)₂ und -OCOR⁶ tragen kann,
   und wobei jedes Alkyl, Alkenyl und Alkinyl geradkettig oder verzweigt sein kann und einen oder mehrere Substituenten, ausgewählt aus Halogen, -OR⁶, -OCOR⁶, -N(R⁶)₂, -NR⁶COR⁶, -NR⁶CON(R⁶)₂, COOR⁶
   oder einem cyclischen Rest, tragen kann,
   oder Salze dieser Verbindungen sowie gegebenenfalls pharmazeutisch übliche Träger, Verdünnungs- oder/und Hilfsmittel enthält.
2. Arzneimittel nach Gegenstand 1,
   **dadurch gekennzeichnet,**
   **dass** es eine oder mehrere Verbindungen der allgemeinen Formel III 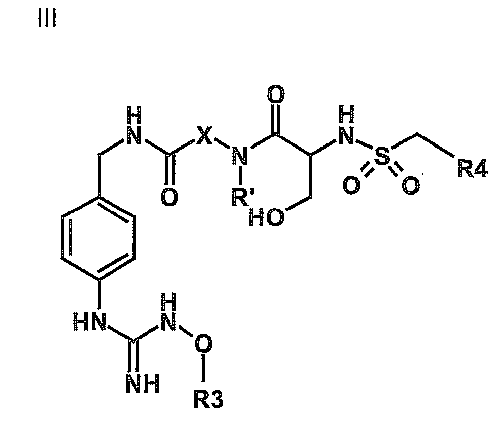 worin
   X, R¹, R³, R⁴ und R⁶ wie in Anspruch 1 definiert sind
   oder Salze dieser Verbindungen enthält.
3. Arzneimittel nach Gegenstand 1 oder 2, worin R⁴ 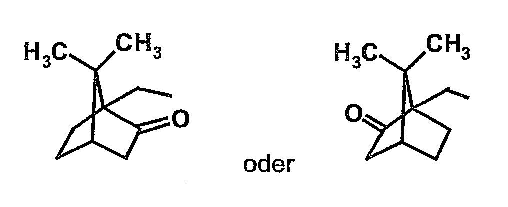 bedeutet.
4. Arzneimittel nach Gegenstand 1 oder 2, worin
   R⁴ einen substituierten oder unsubstituierten C₁-C₃-Alkyl-Aryl-Rest bedeutet, insbesondere einen Benzylrest, der gegebenenfalls in meta-oder para-Position mit Halogen oder/und -NO₂ substituiert sein kann, wobei das Halogen ausgewählt wird aus F, Cl, Br und I.
5. Arzneimittel nach einem der Gegenstände 1 bis 4,
   worin die Verbindungen ausgewählt sind aus
   N-α-(2,4,6-Triisopropylphenylsulfonyl)-3-hydroxyamidino-(L)-phenylalanin-4-ethoxycarbonylpiperazid (WX-671),
   N-α-(2,4,6-Triisopropylphenylsulfonyl)-3-hydroxyamidino-(D)-phenylalanin-4-ethoxycarbonylpiperazid,
   N-α-(2,4,6-Triisopropylphenylsulfonyl)-3-hydroxyamidino-(D,L)-phenylalanin-4-ethoxycarbonylpiperazid,
   N-α-(2,4,6-Triisopropylphenylsulfonyl)-3-hydroxyguanidino-(L)-phenylalanin-4-ethoxy-carbonylpiperazid (WX-683),
   N-α-(2,4,6-Triisopropylphenylsulfonyl)-3-hydroxyguanidino-(D)-phenylalanin-4-ethoxycarbonylpiperazid,
   N-α-(2,4,6-Triisopropylphenylsulfonyl)-3-hydroxyguanidino-(D,L)-phenylalanin-4-ethoxy-carbonylpiperazid,
   N-α-(2,4,6-Triisopropylphenylsulfonyl)-3-hydroxyguanidino-(L)-phenylalanin-4-ethylaminocarbonylpiperazid (WX-685),
   N-α-(2,4,6-Triisopropylphenylsulfonyl)-3-hydroxyguanidino-(D)-phenylalanin-4-ethylaminocarbonylpiperazid,
   N-α-(2,4,6-Triisopropylphenylsulfonyl)-3-hydroxyguanidino-(D,L)-phenylalanin-4-ethylaminocarbonylpiperazid,
   Benzylsulfonyl-(D)-Ser-Gly-(4-hydroxyguanidinobenzyl)amid (WX-678), 4-Chlor-benzylsulfonyl-(D)-Ser-N-Me-Ala-(4-hydroxyguanidinobenzyl) amid,
   4-Chlor-benzylsulfonyl-(D)-Ser-Gly-(4-hydroxyguanidinobenzyl)amid, Benzylsulfonyl-(D)-Ser-N-Me-Gly-(4-hydroxyguanidinobenzyl)amid,
   4-Chlor-benzylsulfonyl-(D)-Ser-Ala-(4-hydroxyguanidinobenzyl)amid,
   oder physiologisch verträglichen Salzen davon.
6. Arzneimittel nach einem der Gegenstände 1 bis 5
   **dadurch gekennzeichnet,**
   **dass** es ein oral verabreichbares Mittel ist.
7. Arzneimittel nach einem der Gegenstände 1 bis 6,
   **dadurch gekennzeichnet,**
   **dass** die Verbindungen in Form der Hydrogensulfate vorliegen.
8. Arzneimittel nach Gegenstand 7,
   worin die Verbindung
   N-α-(2,4,6-Triisopropylphenylsulfonyl)-3-hydroxyamidino-(L)-phenylalanin-4-ethoxycarbonylpiperazinium-Hydrogensulfat (WX-671 ·HSO₄) ist.
9. Verwendung eines Arzneimittels nach einem der Gegenstände 1 bis 8 zur Herstellung einer pharmazeutischen Zusammensetzung für die Bekämpfung von Krankheiten, die mit einer pathologischen Überexpression von Urokinase und/oder Urokinaserezeptor assoziiert sind.
10. Verwendung nach Gegenstand 9 zur Tumorbehandlung oder -prävention.
11. Verwendung nach Gegenstand 10 zur Behandlung oder Prävention der Metastasenbildung.
12. Verwendung nach Gegenstand 11 zur Behandlung von Primärtumoren.
13. Verwendung nach einem der Gegenstände 9 bis 12, worin eine oral verabreichbare Zusammensetzung hergestellt wird.
14. Verwendung nach einem der Gegenstände 9 bis 13, worin die Zusammensetzung in Form von Tabletten, Dragees, Kapseln, Pellets, Lösungen, Emulsionen oder/und Suspensionen hergestellt wird.
15. Verbindungen der Formel I und II 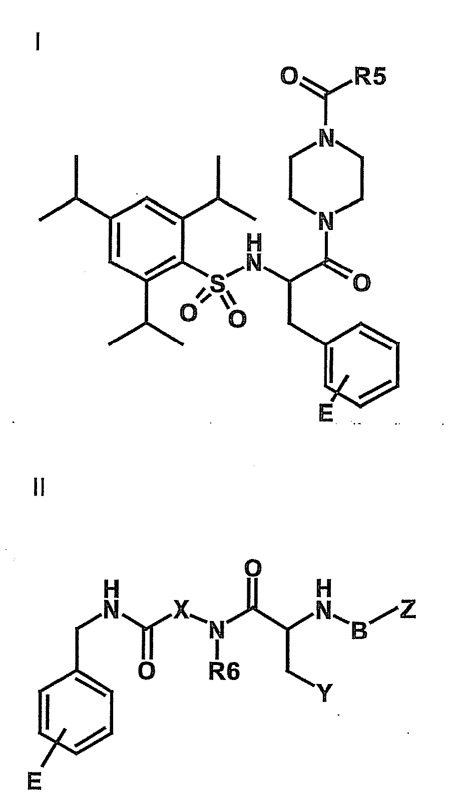 worin E, B, X, Z, Y, R⁵ und R⁶ wie in Anspruch 1 definiert sind.
16. Verbindungen der Formel III 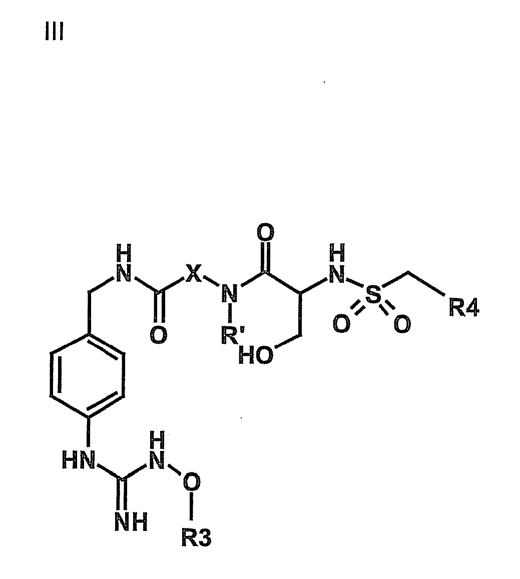 worin X, R¹, R³, R⁴ und R⁶ wie in Anspruch 1 definiert sind.
17. Verbindungen nach Gegenstand 15 oder 16, ausgewählt aus
   N-α-(2,4,6-Triisopropyl-phenylsulfonyl)-3-hydroxyamidino-(L)-phenylalanin-4-ethoxycarbonylpiperazid (WX-671),
   N-α-(2,4,6-Triisopropyl-phenylsulfonyl)-3-hydroxyamidino-(D)-phenylalanin-4-ethoxycarbonylpiperazid,
   N-α-(2,4,6-Triisopropylphenylsulfonyl)-3-hydroxyamidino-(D,L)-phenylalanin-4-ethoxycarbonylpiperazid,
   N-α-(2,4,6-Triisopropylphenylsulfonyl)-3-hydroxyguanidino-(L)-phenylalanin-4-ethoxy-carbonylpiperazid (WX-683),
   N-α-(2,4,6-Triisopropylphenylsulfonyl)-3-hydroxyguanidino-(D)-phenylalanin-4-ethoxycarbonylpiperazid,
   N-α-(2,4,6-Triisopropylphenylsulfonyl)-3-hydroxyguanidino-(D,L)-phenylalanin-4-ethoxy-carbonylpiperazid,
   N-α-(2,4,6-Triisopropylphenylsulfonyl)-3-hydroxy-guanidino-(L)-phenylalanin-4-ethylaminocarbonylpiperazid (WX-685),
   N-α-(2,4,6-Triisopropylphenylsulfonyl)-3-hydroxyguanidino-(D)-phenylalanin-4-ethylaminocarbonylpiperazid,
   N-α-(2,4,6-Triisopropylphenylsulfonyl)-3-hydroxyguanidino-(D,L)-phenylalanin-4-ethylaminocarbonylpiperazid,
   Benzylsulfonyl-(D)-Ser-Gly-(4-hydroxyguanidinobenzyl)amid (WX-678), 4-Chlor-benzylsulfonyl-(D)-Ser-N-Me-Ala-(4-hydroxyguanidinobenzyl) amid,
   4-Chlor-benzylsulfonyl-(D)-Ser-Gly-(4-hydroxyguanidinobenzyl)amid, Benzylsulfonyl-(D)-Ser-N-Me-Gly-(4-hydroxyguanidinobenzyl)amid, 4-Chlor-benzylsulfonyl-(D)-Ser-Ala-(4-hydroxyguanidinobenzyl)amid,
   oder Salzen davon.
18. Verbindungen nach einem der Gegenstände 15 bis 17 in Form der Hydrogensulfate.
19. Verbindungen nach einem der Gegenstände 15 bis 18,
   worin die Verbindung
   N-α-(2,4,6-Triisopropylphenylsulfonyl)-3-hydroxyamidino-(L)-phenylalanin-4-ethoxycarbonylpiperazinium-Hydrogensulfat (WX-671 · HSO₄) ist.
20. Verfahren zur Urokinasehemmung bei Lebewesen durch Verabreichen einer wirksamen Menge mindestens einer Verbindung nach einem der Gegenstände 15 bis 19.
21. Verfahren zur Urokinasehemmung beim Menschen durch Verabreichen einer wirksamen Menge mindestens einer Verbindung nach einem der Gegenstände 15 bis 19.

## Patentansprüche

1. Arzneimittel, das als Wirkstoff eine oder mehrere Verbindungen der allgemeinen Formel II 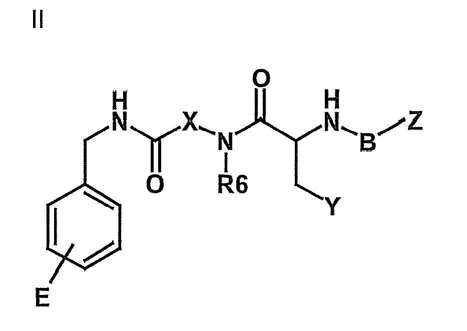 worin
E eine Gruppe aus 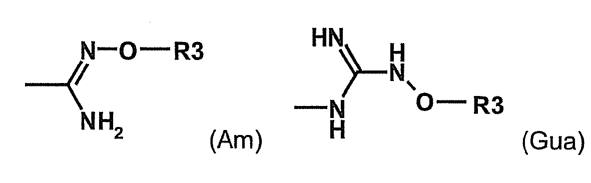 bedeutet,
B -SO₂- oder -CO- bedeutet,
X -NR¹ oder -CHR¹ bedeutet,
Z -R¹, -OR⁴ oder -NH-R⁴ bedeutet,
Y -OR² oder -NHR² bedeutet,
R¹ jeweils unabhängig -H, -C₁-C₆-Alkyl, -C₂-C₆-Alkenyl oder -C₂-C₆-Alkinyl unsubstituiert oder substituiert bedeutet,
R² -H, -OR¹, -COR¹, -CON(R¹)₂,oder -COOR¹ bedeutet,
R³ -H, -C₁-C₆-Alkyl, -C₂-C₆-Alkenyl oder -C₂-C₆-Alkinyl unsubstituiert oder substituiert oder -COR⁶ oder -COOR⁶ oder einen Oligo-oder Poylalkylenoxyrest bedeutet,
R⁴ -H, -C₁-C₆-Alkyl, -C₂-C₆-Alkenyl oder -C₂-C₆-Alkinyl unsubstituiert oder substituiert oder einen cyclischen Rest bedeutet,
R⁶ -H, -C₁-C₆-Alkyl, -C₂-C₆-Alkenyl oder -C₂-C₆-Alkinyl unsubstituiert oder substituiert oder einen cyclischen Rest bedeutet,
wobei jeder cyclische Rest einen oder mehrere Substituenten, ausgewählt aus -C₁-C₃-Alkyl, -C₁-C₃-Alkoxy), Halogen, -OR⁶, =O, -NO₂, -CN, -COOR⁶, -N(R⁶)₂, -NR⁶COR⁶, -NR⁶CON(R⁶)₂ und -OCOR⁶ tragen kann,
und wobei jedes Alkyl, Alkenyl und Alkinyl geradkettig oder verzweigt sein kann und einen oder mehrere Substituenten, ausgewählt aus Halogen, -OR⁶, -OCOR⁶, -N(R⁶)₂, -NR⁶COR⁶, -NR⁶CON(R⁶)₂, COOR⁶ oder einem cyclischen Rest, tragen kann,
oder Salze dieser Verbindungen sowie gegebenenfalls pharmazeutisch übliche Träger, Verdünnungs- oder/und Hilfsmittel enthält.

2. Arzneimittel nach Anspruch 1,
**dadurch gekennzeichnet,**
**dass** es eine oder mehrere Verbindungen der allgemeinen Formel III 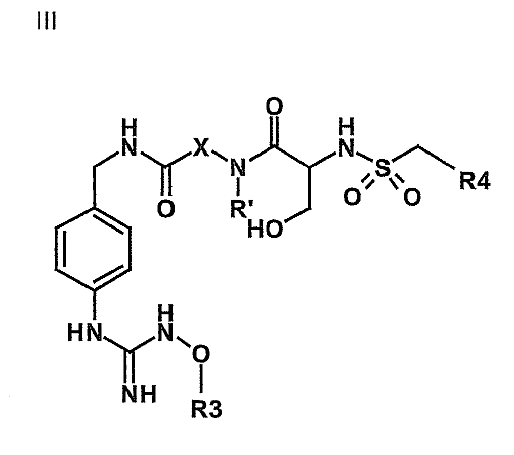 worin
X, R¹, R³, R⁴ und R⁶ wie in Anspruch 1 definiert sind
oder Salze dieser Verbindungen enthält.

3. Arzneimittel nach Anspruch 1 oder 2, worin R⁴ 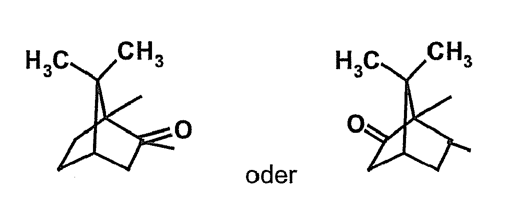 bedeutet.

4. Arzneimittel nach Anspruch 1 oder 2, worin
R⁴ einen substituierten oder unsubstituierten C₁-C₃-Alkyl-Aryl-Rest bedeutet, insbesondere einen Benzylrest, der gegebenenfalls in meta-oder para-Position mit Halogen oder/und -NO₂ substituiert sein kann, wobei das Halogen ausgewählt wird aus F, Cl, Br und I.

5. Arzneimittel nach einem der Ansprüche 1 bis 4,
worin die Verbindungen ausgewählt sind aus
Benzylsulfonyl-(D)-Ser-Gly-(4-hydroxyguanidinobenzyl)amid (WX-678), 4-Chlor-benzylsulfonyl-(D)-Ser-N-Me-Ala-(4-hydroxyguanidinobenzyl) amid,
4-Chlor-benzylsulfonyl-(D)-Ser-Gly-(4-hydroxyguanidinobenzyl)amid, Benzylsulfonyl-(D)-Ser-N-Me-Gly-(4-hydroxyguanidinobenzyl)amid, 4-Chlor-benzylsulfonyl-(D)-Ser-Ala-(4-hydroxyguanidinobenzyl)amid,
oder physiologisch verträglichen Salzen davon.

6. Arzneimittel nach einem der Ansprüche 1 bis 5
**dadurch gekennzeichnet,**
**dass** es ein oral verabreichbares Mittel ist.

7. Arzneimittel nach einem der Ansprüche 1 bis 6,
**dadurch gekennzeichnet,**
**dass** die Verbindungen in Form der Hydrogensulfate vorliegen.

8. Verwendung eines Arzneimittels nach einem der Ansprüche 1 bis 7 zur Herstellung einer pharmazeutischen Zusammensetzung für die Bekämpfung von Krankheiten, die mit einer pathologischen Überexpression von Urokinase und/oder Urokinaserezeptor assoziiert sind.

9. Verwendung nach Anspruch 8 zur Tumorbehandlung oder -prävention, insbesondere zur Behandlung oder Prävention der Metastasenbildung oder zur Behandlung von Primärtumoren.

10. Verwendung nach einem der Ansprüche 8 bis 9, worin eine oral verabreichbare Zusammensetzung hergestellt wird.

11. Verwendung nach einem der Ansprüche 8 bis 10, worin die Zusammensetzung in Form von Tabletten, Dragees, Kapseln, Pellets, Lösungen, Emulsionen oder/und Suspensionen hergestellt wird.

12. Verbindungen der Formel II 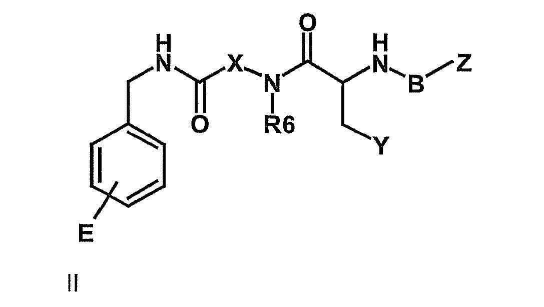 worin E, B, X, Z, Y, und R⁶ wie in Anspruch 1 definiert sind.

13. Verbindungen der Formel III 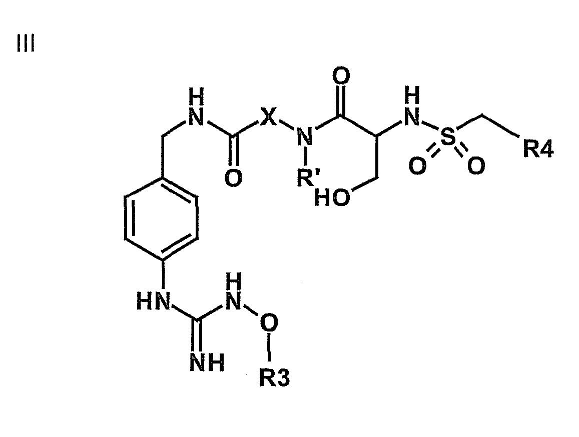 worin X, R¹, R³, R⁴ und R⁶ wie in Anspruch 1 definiert sind.

14. Verbindungen nach Anspruch 12 oder 13, ausgewählt aus
Benzylsulfonyl-(D)-Ser-Gly-(4-hydroxyguanidinobenzyl)amid (WX-678), 4-Chlor-benzylsulfonyl-(D)-Ser-N-Me-Ala-(4-hydroxyguanidinobenzyl) amid,
4-Chlor-benzylsulfonyl-(D)-Ser-Gly-(4-hydroxyguanidinobenzyl)amid, Benzylsulfonyl-(D)-Ser-N-Me-Gly-(4-hydroxyguanidinobenzyl)amid, 4-Chlor-benzylsulfonyl-(D)-Ser-Ala-(4-hydroxyguanidinobenzyl)amid,
oder Salzen davon.

15. Verbindungen nach einem der Ansprüche 12 bis 14 in Form der Hydrogensulfate.
